(19) Europäisches Patentamt

European Patent Office

Office européen des brevets

(11) **EP 1 374 812 A1**

(12) **EUROPEAN PATENT APPLICATION**

(43) Date of publication:
**02.01.2004 Bulletin 2004/01**

(51) Int Cl.[7]: **A61F 13/02**, A61F 13/00,
C08J 9/34

(21) Application number: **02014474.7**

(22) Date of filing: **28.06.2002**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **Biopol Co., Ltd.
Hwasung-city, Kyunggi-do, 445-924 (KR)**

(72) Inventors:
• **Park, Myung-Hwan
Gangnam-gu Seoul 135-270 (KR)**

• **Lee, Soo-Chang
Seoul 136-827 (KR)**
• **Kim, Hyung-Jung
Whasung-city, Kyunggi-Do 445-890 (KR)**
• **Kang, Sun-Ae
Dongan-gu Anyang-City
Kyunggi-do 431-080 (KR)**

(74) Representative: **Dörries, Hans Ulrich, Dr.
Dörries, Frank-Molnia & Pohlman,
Triftstrasse 13
80538 München (DE)**

(54) **Multilayered microporous foam dressing and method for manufacturing the same**

(57) Disclosed is a multilayered microporous foam dressing 1-7mm thick, having a three-ply structure consisting of an outer protective film layer 10-90µm thick, an intermediate absorbent foam layer containing numerous open cells with an average diameter of 80-400µm, and a wound-contacting layer 0.1-200µm thick with numerous pores having an average diameter of 60µm. A method for preparing the dressing is also disclosed. 40-75wt% of a polyurethane prepolymer, 15-45wt% of a foaming agent, 5-35wt% of a crosslinking agent, and 0.5-15wt% of additives, including a surfactant, a humectant, a releasing agent, antibacterial agents, dyes, cell growth factors, etc., are mixed by stirring, and the mixture is injected into a mold coated with the outer protective film layer and allowed to be expanded therein.

**Description**

BACKGROUND OF THE INVENTION

1. Field of the Invention

The present invention relates, in general, to a foam dressing for use in covering and protecting wounds and, more particularly, to a moist occlusive dressing capable of improving wound healing effects. Also, the present invention is concerned with a method for manufacturing such a foam dressing.

2. Description of the Prior Art

The skin serves many purposes: it serves as a barrier to the environment such as pressure or friction, noxious chemicals, heat, cold and harmful microorganisms. Also, the skin helps regulate body temperature through action of sweat glands and metabolism, as well as being responsible for the sense of touch. In addition, it protects us from water loss, friction wounds, and impact wounds.

Wounds, whether the skin is damaged, burned, ulcerated or otherwise traumatized, cause the skin to lose its full function, at least at the wound area and, in the worst case, may be fatal to the patient. In addition, wounds leave scars after the treatment, subjecting the patient to a secondary pain.

Beginning any time there is an insult to the body that causes a break in the skin or tissue, the wound healing process is generally defined as a cascade, with overlapping phases including inflammatory phase, proliferative phase, and maturation phase. The inflammation results from the initial response to injury. With the increase in blood from vasodilation there is an increase in capillary permeability allowing blood plasma to leak into the surrounding tissue, which produces inflammatory exudates. Neutrophils and monocytes are attracted to the site of injury by a variety of chemotactic factors produced in response to tissue damage. Monocytes undergo a phenotypic change to become activated macrophages. The macrophages produce various growth factors (PDGF, TGF-β, EGF, FGF, etc.) that can initiate, accelerate or modify the healing process. They also produce cytokines (IL-1, IL-6, IL-8, TNF, etc.) that act as messengers between individual cells. The formation of new blood vessels occurs with the release of angiogenic growth factors which stimulate endothelial cell proliferation and stimulates the growth of new blood vessels. Classic signs of the proliferative phase include granulation, fibrous tissue formation, collagen deposition, epithelialization, and contraction of the wound, for which the growth factors and cytokines are responsible. The final phase of wound healing is maturation or remodeling. In this phase, the proliferated cells secrete growth factors by themselves. Contraction, a reduction in blood supply to the site, and scar formation occur in the maturation phase, along with collagen turnover and reorganization. The wound loses mass while gaining resilience and strength. During this phase the type III collagen, a soft gelatinous collagen laid down in the proliferation phase, is replaced with a more highly organized collagen type I. The differentiation of collagen is a dynamic process and although it takes place predominantly during the maturation phase, it may continue to be remodeled indefinitely.

In order to help the healing process, the wound site is required to maintain a moist, bacteria-free environment. Also, the removal of debris and the absence of necrotized tissues promote the healing process. Other factors which positively affect wound healing include high oxygen concentration, rich cell growth factors, and the prevention of the maceration of normal skin around the wound bed. Accordingly, ideal dressings are those which meet the following requirements: maintenance of an appropriate moist environment at the contact face of the wound, rapid absorption of exudates from the wound, easy attachment to and removal from the wound, maintenance of high oxygen tension and appropriate vapor permeability, heat insulation to keep the wound site at 32-35 °C, defense against bacterial infection, prevention of the leakage of cell growth factors, harmlessness to the body, excellent mechanical properties, and economic advantages.

Conventional gauze dressings can well absorb exudates from wounds, but cannot defend against bacterial infection nor provide moist conditions to wounds, thus retarding the wound healing. In addition, because gauze dressings are apt to hold on fast to wounds, they are not easily changed with fresh ones and may damage newly regenerated tissues with accompaniment of pain upon their removal. Further, large quantities of exudates generated at the early stage of the wound healing process force conventional gauze dressings to be changed many times a day.

Thus far, there have been developed a variety of closed dressings that are improved in comparison with gauze dressings. However, closed dressings in current use are very expensive and have difficulty in controlling their absorption capacity and moisture permeability. With these problems, closed dressings are not applied to a broad spectrum of wounds, but mainly to specific wounds.

On the whole, dressings in current use may be broken down into the following five types: film, hydrocolloid, hydrogel, non-woven, and polyurethane foam. Among them, hydrocolloid-, hydrogel-, and polyurethane foam-type dressings show particularly excellent healing effects.

U. S. Pat. Nos. 5,503,847 and 5,830,932 disclose hydrocolloid type dressings that comprise a hydrocolloid layer for relieving external impact and absorbing exudates and a film layer for defending against the infiltration of bacteria and impurities. The hydrocolloid type dressings form gel upon contact with wound exudates to keep the wound bed moist, without desiccating or dehydrating the wound bed, and to produce a wound environment suitable for healing. Also, the hydrocolloid type dressings provide a weakly acidic environment for a long time period to prevent the wound from being injured and to promote the growth of cells. However, the hydrocolloid type dressings are not suitable for healing of wounds that produce large quantities of exudates because of their deficiency in moisture permeability and exudate-absorbing capacity. The gel is likely to partially remain on the wound bed on removal. In this case, the remainder serves as a nutritive source for bacteria and thus, an additional work is needed to remove it.

Hydrogel-type dressings comprising an impermeable polymeric film layer coated with a hydrogel material are found in U. S. Pat. Nos. 5,501,661 and 5,489,262. The polymeric film layer functions to prevent the hydrogel from being dehydrated or desiccated, while the hydrogel absorbs exudates in contact with the wound bed and provides a moist environment to promote wound healing. However, the hydrogel-type dressings are unsuitable for use in absorbing large quantities of exudates owing to their poor moisture permeability and, when being swollen with water for a long period of time, the dressing substance collapses, causing the maceration of normal skin.

U. S. Pat. Nos. 5,445,604 and 5,065,752 introduce hydrophilic polyurethane foam dressings in which a polyurethane foam material is sandwiched between a wound facing layer and an outer film, the wound facing layer being perforated to absorb wound exudate. However, the poor moisture permeability of the non-perforated outer film makes it difficult to apply the dressings for a long period of time. Further, freshly generated tissue grows into the gel through the apertures present in the facing layer, so that injury is apt to be caused on removal.

A multilayered wound dressing is disclosed in U. S. Pat. No. 5,445,604, comprising an outer protective layer, an intermediate, absorbent layer, a molecular filtration membrane, a wound-contacting layer, and an adhesive coating layer. In this structure, the molecular filtration membrane acts to rapidly remove wound exudates into the intermediate absorbent layer and retain high concentrations of healing factors such as cytokines (e.g., PDGF, TGF-β, EGF, FGF, IL-1, IL-6, IL-8, TNF and others), glycosaminoglycans(hyaluronic acid, chondroitin sulfates, heparin, dermatan, dermatan sulfates, etc.), and proteins (proteases, etc.) in the wound bed to promote wound healing. Forming a gel in contact with wound exudates, the wound-contacting layer can be easily removed. However, the multilayered wound dressing has difficulty in being applied for general uses because of its high cost. Further, it requires a complicated process for manufacture.

As described above, the problems of prior art closed dressings can be summarized into low absorptivity, high adherence to wound, poor physical properties, and high price, and being unsuitable for use in wounds producing large exudates.


SUMMARY OF THE INVENTION

Leading to the present invention, the intensive and thorough research into a wound dressing, conducted by the present inventors aiming to overcome the above-mentioned problems, resulted in the finding that a triple layer structure consisting of a non-porous outer protective film layer for defending against the infiltration of impurities and bacteria and protecting against the leakage of wound exudates, an intermediate absorbent foam layer, having numerous open cells with a average diameter of 80 to 400 μm, for absorbing wound exudates and allowing the permeation of moisture, and a wound-contacting layer containing micropores with an average diameter less than 60 μm, for retaining healing factors, including growth factors, cytokines, glycosaminoglycan, proteins, etc., can overcome prior art problems and promote wound healing at higher efficiency with minimal scar formation.

It is therefore an object of the present invention to overcome the problems encountered in prior arts and to provide a foam dressing having the multilayered structure with which an improved wound healing environment is provided.

It is another object of the present invention to provide a foam dressing which promotes wound healing at an improved rate with formation of minimal cicatrice.

It is still another object of the present invention to provide a method for manufacturing the foam dressing with ease.

In accordance with an aspect of the present invention, there is provided a multilayered foam dressing 1-7 mm thick, having a three-ply structure consisting of an outer protective film layer 10-90 μm thick, an intermediate absorbent foam layer containing numerous open cells with an average diameter of 80-400 μm, and a wound-contacting layer 0.1-200 μm thick with numerous pores having an average diameter less than 60 μm.

In accordance with another aspect of the present invention, there is provided a method for manufacturing a multilayered foam dressing, comprising the steps of: mixing 40-75 wt% of a polyurethane prepolymer, 15-45 wt% of a foaming agent, 5-35 wt% of a crosslinking agent, and 0.5-15 wt% of additives comprising a surfactant, a humectant, a releasing agent, an antibacterial agent, and pigments, said polyurethane prepolymer being prepared from molar ratios of 1-3 : 0.15-0.5 diisocyanate: polyol, and expanding the mixture at mold temperature of 30-60 °C.

BRIEF DESCRIPTION OF THE DRAWINGS

Fig. 1 is a schematic cross sectional view showing the three-ply structure of the foam dressing of the present invention, which consists of an outer protective film layer, an intermediate absorbent layer, and a microporous wound-contacting layer.

Fig. 2 is scanning electron microphotographs showing the surface of an outer protective film layer, the surface of a wound-contacting layer, and the cross section of an intermediate absorbent foam layer.

DETAILED DESCRIPTION OF THE INVENTION

The application of the preferred embodiments of the present invention is best understood with reference to the accompanying drawings.

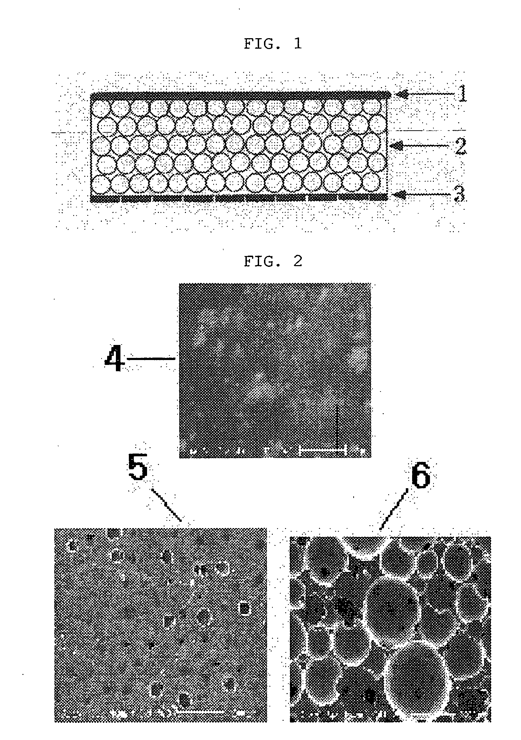

Referring to Fig. 1, there is a schematic cross section showing the multilayered foam dressing of the present invention. As seen, the multilayered foam dressing has a three-ply structure comprising an outer protective film layer 1, an intermediate absorbent foam layer 2, and a wound-contacting layer 3. With a thickness of from 1 to 7 mm, the multilayered foam dressing can be produced in various shapes according to purposes.

Ranging in thickness from 10 to 90 $\mu$m, the outer protective film layer 1 has the function of protecting against the infiltration of impurities and bacteria and preventing the leakage of wound exudates, and also has high moisture permeability.

Responsible for the high absorption of wound exudate and high moisture permeability of the multilayered foam dressing, the intermediate absorbent foam layer 2 contains a large number of open cells, each 80-400 $\mu$m in diameter, at an amount of 50 to 80 % by volume, and its specific gravity is within the range of 0.1 to 0.4.

Being 0.1-200 $\mu$m thick, the wound-contacting layer 3 is characterized by the existence of numerous micropores thereon and the ability to retain healing factors such as cell growth factors, cytokines, glycosaminoglycans, proteins, etc., in the wound bed. Occupying 5-50 % of the total surface area of the wound-contacting layer 3, the micropores have a diameter less than 60 $\mu$m on average and make the wound-contacting layer 3 non-adherent to the wound bed, as well as serving to concentrate the healing factors on the wound bed. The micropores comprise a structure opening into the exterior and a structure separated from the exterior by being covered with a thin film membrane(cell window).

In Fig. 2, scanning electronic photographs 4, 5 and 6 which show the surface of the outer protective film layer 1, the surface of the wound-contacting layer 3, and the cross section of the intermediate absorbent foam layer 2, respectively.

The outer protective film layer 1 of the foam dressing of the present invention is preferably prepared by using a resin having high hydrophile property and high moisture permeability. Examples of the resin include synthetic polymers, which may be exemplified by polyurethane, polyethylene, silicon rubber, synthetic rubber, polyglycolic acid, polylactic acid and its copolymers, polyvinyl alcohol, polyvinylpyrrolidone, etc., natural polymers, which may be exemplified by collagen, gelatin, hyaluronic acid, sodium alginate, chitin, chitosan, fibrin, cellulose, etc., synthetic polymers from the natural sources, or synthetic polymers modified from the natural polymers. Most preferable is polyurethane. The outer protective film layer 1, preferably, is in a dry film form, which may be prepared by dissolving polyurethane in a suitable solvent, coating a release paper with the dissolved polyurethane to a suitable thickness, and then volatilizing the solvent.

Also, the intermediate absorbent foam layer 2 and the wound-contacting layer 3 of the foam dressing of the present invention may be prepared from synthetic polymers, which may be exemplified by polyurethane, polyethylene, silicon rubber, synthetic rubber, polyglycolic acid, polylactic acid and its copolymers, polyvinyl alcohol, polyvinylpyrrolidone, etc., natural polymers, which may be exemplified by collagen, gelatin, hyaluronic acid, sodium alginate, chitin, chitosan, fibrin, cellulose, etc., synthetic polymers from the natural polymers, or combinations thereof. Most preferable is polyurethane. When using polyurethane, 40-75 wt% of a polyurethane prepolymer, which can be prepared by the reaction of at least one polyol with diisocyanate, 15-45 wt% of a foaming agent, 5-35 wt% of a crosslinking agent, and 0.5-15 wt% of additives, including a surfactant, a humectant, a releasing agent, antibacterial agents, pigments, cell growth factors, etc., are mixed by stirring, and the mixture is injected into a mold and allowed to be expanded therein. While being maintained at 30-60 °C, the mold is opened 5-10 min after the injection.

For the expansion molding, a highly breathable polyurethane dry film is fixed on only one side of the mold to make the resulting outer protective film layer 1 with a non-porous structure. As used herein, the term "non-porous" means that pores are undetectable under a scanning electron microscope and a transmission electron microscope as well and pores over 20 nm across do not exist in the non-swollen state, excepting closed cells, pin holes, etc., that may be produced in the film during a preparation process.

For the preparation of the polyurethane prepolymer, diisocyanate is reacted to polyol at molar ratios of 1-3 : 0.15-0.5. Examples of the diisocyanate useful in the present invention include isoporone diisocyanate, 2,4-toluenediisocyanate and its isomers, diphenylmethanediisocyanate, hexamethylenediisocyanate, lysine diisocyanate, trimethylhexamethylene diisocyanate, 2,2-bis-4'-propaneisocyanate, 6-isopropyl-1,3-phenyldiisocyanate, bis(2-isocyanateethyl)fumarate, 3,3'-dimethyl-4,4'-diphenylmethanediisocyanate, 1,6-hexanediisocyanate, 4,4'-biphenylenediisocyanate, 3,3'-dimethylphenylenediisocyanate, p-phenylenediisocyanate, m-phenylenediisocyanate, 1,5-naphthalenediisocyanate, 1,4-xylenediisocyanate, and 1,3-xylenediisocyanate with preference for diphenylmethanediisocyanate, 2,4-toluenediisocyanate and its isomers, p-phenylenediisocyanate, isoporonediisocyanate, and hexamethylenediisocyanate. As for the polyol, it may be prepared by mixing an ethylene oxide/propylene oxide random copolymer which contains at least three hydroxyl groups and ranges in molecular weight from 3,000 to 6,000 with an ethylene oxide content of 50 to 80 %, with a polypropylene glycol which contains at least two hydroxyl groups with a molecular weight of 1,000 to 4,000, in weight ratios of 30:70. Preferably, the ethylene oxide/propylene oxide random copolymer which contains at least three hydroxyl groups and ranges in molecular weight from 3,000 to 6,000 with an ethylene oxide content of 50 to 80 %, is used alone.

Useful as the foaming agent are chlorofluorocarbon (CFC-141b), methylene chloride and distilled water. Distilled water is preferable.

To function as the crosslinking agent, at least two hydroxyl groups in a molecule are required. Examples of the crosslinking agent useful in the present invention include 1, 3-butanediol, 1,4-butanediol, 1,5-pentanediol, 1,6-hexanediol, neophentylglycol, propyleneglycol, ethylene glycol, polyethylene glycol with a molecular weight of 200 to 2,000, glycerol, trimethylolethane, trimethylolpropane, pentaerythritol, sorbose, sorbitol, and combinations thereof with preference for glycerol, sorbitol, polyethyleneglycol with a molecular weight of 200-2,000, and trimethylolpropane.

The surfactant useful in the present invention may be exemplified by L-62, L-64, P-84, P-85, P-105, F-68, F-87, F-88, F-108, and F-127, all of which are kinds of ethylene oxide/propylene oxide block copolymers, manufactured by BASF, Germany, and L-508, L-5305, L5302 and L-3150, which are all silicone-based, manufactured by Osi, U.S.A.

To serve as a humectant and wound healing promoter, hyaluronic acid, keratan, collagen, dermatan sulfate, heparin, heparan sulfate, sodium alginate, pectin, xanthan gum, Guar gum, karaya gum, sodium carboxymethylcellulose, chondroitin sulfate, 3-aminopropyldihydrogen phosphate, chitin, chitosan, gelatin, locust bean gum, oligosaccharides thereof, or combinations thereof may be used.

As the growth factor, platelet-derived growth factor (PDGF), transforming growth factor beta (TGF-$\beta$), epidermal growth factor (EGF), fibroblast growth factor (FGF) may be used, alone or in combination.

Known as a silicon-based surfactant, L-45, L-580, L-3002, or L-5309, all manufactured by Osi, may be used as a releasing agent in the present invention.

Any antibacterial agent, if pharmaceutically acceptable, may be used in the present invention. Examples include gluconate chlorohexidin, acetate chlorohexidin, hydrochloride chlorohexidin, silver sulfur diazine, povidone iodine, benzalconium chloride, furazin, idocaine, hexachlorophene, chlorotetracycline, neomycin, penicillin, gentamycin, and acrinol.

A better understanding of the present invention may be obtained in light of the following examples which are set forth to illustrate, but are not to be construed to limit the present invention.

SYNTHESES EXAMPLE 1

Preparation of a isocyanate-capped polyurethane prepolymer was carried out as follows.

Into a 3 liter round-bottom flask were added 242.8 g of TDI (toluenediisocyanate), and then heated to 60 °C. Afterwards, while 1257.5 g of TR-705, which is an ethylene oxide/propylene oxide random copolymer having three hydroxyl groups with an ethylene oxide content of 75 %, manufactured by Korea Polyol, was added in many small installments, the reactants were reacted for 7 hours to prepare a polyurethane prepolymer until the theoretical NCO% reached 5.7. During the reaction, samples were taken to measure NCO% according to a titration method using an n-butylamine standard solution.

SYNTHESES EXAMPLE 2

Preparation of a isocyanate-capped polyurethane prepolymer was carried out as follows.

Into a 3 liter round-bottom flask were added 248.2 g of TDI (toluenediisocyanate), and then heated to 60 °C. Afterwards, while 816.3 g of TR-705, which is an ethylene oxide/propylene oxide random copolymer having three hydroxyl groups with an ethylene oxide content of 75 %, manufactured by Korea Polyol, and 435.4 g of GP-4000, which is a polypropyleneglycol having three hydroxyl groups, were added in many small installments, the reactants were reacted for 7 hours to prepare a polyurethane prepolymer until the theoretical NCO% reached 5.7. During the reaction, samples were taken to measure NCO% according to a titration method using an n-butylamine standard solution.

EXAMPLE 1

An outer protective film layer was prepared as follows. 18 g of polyurethane elastomer was added into a DMF/MEK (40/60) solvent, and the mixture was then heated to 60 °C with stirring, resulting in a polyurethane solution in a colorless, transparent liquid state. A release paper, treated with silicon and having a constant thickness by attaching a tape to its edges, was uniformly coated with the polyurethane solution using a film coater, following by drying in an oven at 100 °C for over 1 hour. The obtained polyurethane films were 15, 30, and 50 μm in thickness.

EXAMPLE 2

To 57.1 wt% of the polyurethane prepolymer prepared in Syntheses Example 1, 27.2 wt% of distilled water as a foaming agent, and 13.6 wt% of glycerin as a crosslinking agent were added, along with additives comprising 0.5 wt% of F-127 (BASF), 0.9 wt% of L-64, and 0.7 wt% of sodium alginate. After being stirred at 4,000 rpm for 5 sec, the mixture was injected into a mold whose one side had been treated with the polyurethane film 15 μm thick prepared in Example 1. Expansion was conducted at mold temperature of 35 °C for 10 min and the mold was opened to give a hydrophilic polyurethane foam dressing having a density of 0.21 g/cm³.

The hydrophilic polyurethane foam dressing was measured for physical properties in the following processes and the measured results are given in Table 1, below.

1. Mechanical properties (tensile; strength, elongation, modulus)

Measurements were made by use of a tensile tester, such as that manufactured by Instron Corporation, identified as Universal Test Machine, according to JIS-K-6401.

2. % Absorptivity

The hydrophilic polyurethane foam dressing was cut to a size of 3 cm x 3 cm and the sample piece was weighed (A). It was immersed in distilled water at 25 °C for 24 hours, followed by the removal of moisture from the surface with dust-free paper. Its wet weight was measured (B). %. Absorptivity of the hydrophilic polyurethane foam dressing was calculated by means of the following equation.

$$\% \; Absorptivity = \frac{B - A}{A} \times 100$$

3. Moisture permeability

Using an incubator, the hydrophilic polyurethane foam dressing was measured according to KS M 6886. In this regard, the incubator was kept at a temperature of 37.5 °C at a relative humidity of 90 %. Moisture permeability was calculated by means of the following equation.

$$P = \frac{A}{S}$$

$$A = ( \, (a_1 \text{-} a_0) + (a_2 \text{-} a_1) + (a_3 \text{-} a_2) \, ) \, /3$$

where

P is moisture permeability (g/m²/24hr)
A is an average increment for 1 hour (g)
S is a specimen area through which moisture permeation occurs (m²)
$a_0$ is the specimen weight measured after 1 hour
A $a_1$, $a_2$ and $a_3$ are the specimen weights measured after 2, 3 and 4 hours, respectively.

4. Cell and pore sizes, and film thickness

The hydrophilic polyurethane foam dressing was measured for cell size, pore size, and film thickness using a scanning electron microscope.

5. Assay for toxicity to cells

The hydrophilic polyurethane foam dressing was assayed for toxicity to cells according to ISO 10993-5. For this, mouse fibroblast cell 3T3 was obtained from National Institutes of Health and subcultured. After inoculation at a cell density of $2x10^4$ cell/cm$^2$ in RPMI-1640 (Rosewell Park Memorial Institute-1640) supplemented with 10% fetal calf serum (FCS) on 12-well plastic dishes, the 3T3 cells were cultured at 37 °C for 4 days in a 5% $CO_2$ atmosphere. Thereafter, the cells were further cultured for 1, 3 and 5 days while being in direct contact with the hydrophilic polyurethane foam dressing, followed by harvesting the cells with a trypsin/EDTA (ethylene diamine tetraacetic acid) solution. Viable cells were quantified with the aid of a hematocytometer after dyeing with 0.4 % trypan blue.

6. Assay for wound healing effect

Rats which were 6-8 weeks aged on average, weighing 350-300 g were used to observe the wound healing effects of the hydrophilic polyurethane foam dressing. To this end, a dermal lesion with a size of 4 cm x 4 cm was made on the back of each rat which had been anaesthetized by abdominal injection with Rembutal, and then subjected to dressing. At 1, 2 and 3 weeks after the dressing, the wound was examined for size change, tissue separation upon changing of dressing, and histochemistry, to determine the wound healing effect of the hydrophilic polyurethane foam dressing.

EXAMPLE 3

Using the same procedure as in Example 2, a hydrophilic polyurethane foam dressing was prepared, excepting one side of a mold being treated with the polyurethane 30 μm thick prepared in Example 1, in order to obtain a outer protective film layer different in thickness from one prepared in Example 2. The resulting hydrophilic polyurethane foam dressing was measured for physical properties using the same processes as in Example 2, and the measured results are given in Table 1, below.

EXAMPLE 4

Using the same procedure as in Example 2, a hydrophilic polyurethane foam dressing was prepared, excepting one 'side of a mold being treated with the polyurethane 50 μm thick prepared in Example 1, in order to obtain an outer protective film layer different in thickness from one prepared in Examples 2 or 3.
The resulting hydrophilic polyurethane foam dressing was measured for physical properties using the same processes as in Example 2, and the measured results are given in Table 1, below.

EXAMPLE 5

To 60 wt% of the polyurethane prepolymer prepared in Syntheses Example 2, 27.9 wt% of distilled water as a foaming agent, and 10 wt% of glycerin as a cross linking agent were added, along with additives comprising 0.5 wt% of F-127 (BASF), 0.9 wt% of L-64, and 0.7 wt% of sodium alginate. After being stirred at 4,000 rpm for 5 sec, the mixture was injected into a mold whose one side had been treated with the polyurethane film 15 μm thick prepared in Example 1. Expansion was conducted at 50 °C for 7 min and the mold was opened to give a hydrophilic polyurethane foam dressing having a density of 0.21 g/cm$^3$.
The resulting hydrophilic polyurethane foam dressing was measured for physical properties using the same processes as in Example 2, and the results are given in Table 1, below.

EXAMPLE 6

Using the same procedure as in Example 5, a hydrophilic polyurethane foam dressing was prepared, excepting one side of a mold being treated with the 30 μm thick polyurethane film prepared in Example 1, in order to obtain a outer protective film layer different in thickness from one prepared in Exmaple 5.
The resulting hydrophilic polyurethane foam dressing was measured for physical properties using the same processes as in Example 2, and the measured results are given in Table 1, below.

EXAMPLE 7

Using the same procedure as in Example 5, a hydrophilic polyurethane foam dressing was prepared, excepting one side of a mold being treated with the 50 μm thick polyurethane film prepared in Example 1, in order to obtain a outer protective film layer different in thickness from one prepared in Exmaple 5 or 6.
The resulting hydrophilic polyurethane foam dressing was measured for physical properties using the same processes as in Example 2, and the results are given in Table 1, below.

EXAMPLE 8

Using the same procedure as in Example 2, a hydrophilic polyurethane foam dressing was prepared, except for the addition of 0.9 wt% of F-127 and 0.6 wt% of L-64 to modify the content of surfactants.
The resulting hydrophilic polyurethane foam dressing was measured for physical properties using the same processes as in Example 2, and the results are given in Table 1, below.

EXAMPLE 9

Using the same procedure as in Example 2, a hydrophilic polyurethane foam dressing was prepared, except for the addition of 1.2 wt% of F-127 and 0.3 wt% of L-64 to modify the content of surfactants.
The resulting hydrophilic polyurethane foam dressing was measured for physical properties using the same processes as in Example 2, and the results are given in Table 1, below.

EXAMPLE 10

Using the same procedure as in Example 9, a hydrophilic polyurethane foam dressing was prepared, excepting that expansion was conducted at mold temperature of 60 °C for 5 min.
The resulting hydrophilic polyurethane foam dressing was measured for physical properties using the same processes as in Example 2, and the measured results are given in Table 1, below.

COMPARATIVE EXAMPLE 1

Allevyn, a commercially available polyurethane foam dressing from Smith & Nephew, was used. Its physical properties were measured in the same processes as in Example 2 and the results are given in Table 1, below.

COMPARATIVE EXAMPLE 2

DuoDerm, a commercially available hydrocolloid dressing from ConvaTec, was used. Its physical properties were measured in the same processes as in Example 2 and the results are given in Table 1, below.

TABLE 1

| Exam. No. | Outer Protective Film Thick. (µm) | Elong. (%) | 100% Modulus (kgf/mm$^2$) | Absorp. (%) | Average Pore Size of Wound-contacting Layer (µm) | Moist. Permeab. (g/ m$^2$/24hr) | Non-Toxicity | Healing Effect | Wound Non-Adherence |
|---|---|---|---|---|---|---|---|---|---|
| Physical Properties of Hydrophilic Polyurethatne Foam Dressing | | | | | | | | | |
| 2 | 15 | 420 | 0.009 | 950 | 40 | 2,700 | ◎ | ◎ | ◎ |
| 3 | 30 | 450 | 0.010 | 950 | 40 | 1,900 | ◎ | ◎ | ◎ |
| 4 | 50 | 450 | 0.012 | 1,030 | 40 | 1,500 | ◎ | ◎ | ◎ |
| 5 | 15 | 420 | 0.008 | 380 | 55 | 2,500 | ◎ | ◎ | ◎ |
| 6 | 30 | 430 | 0.010 | 400 | 55 | 1,800 | ◎ | ◎ | ◎ |
| 7 | 50 | 440 | 0.012 | 400 | 55 | 1,300 | ◎ | ○ | ◎ |
| 8 | 15 | 420 | 0.009 | 980 | 90 | 2,700 | ◎ | ◎ | 0 |
| 9 | 15 | 440 | 0.009 | 980 | 160 | 2,800 | ◎ | ○ | 0 |
| 10 | 15 | 450 | 0.010 | 1,050 | 210 | 3,000 | ◎ | ○ | 0 |
| C.1 | - | 390 | 0.012 | 630 | ≥ 500 | 1,400 | ○ | Δ | Δ |
| C.2 | - | - | - | 250 | - | 200 | ○ | ○ | × |
| Note ◎ : Excellent, ○ : Good, Δ : Moderate, × : poor | | | | | | | | | |

EP 1 374 812 A1

As apparent from the data of Examples 2, 3 and 4, when the thickness of the outer protective film layer reduces, moist permeability comes into increase. Thus, regulating the thickness of the outer protective film layer can easily control entire moist permeability of foam dressing.

The data of Examples 5 and 6 demonstrate that the content ratio between the hydrophobic and hydrophilic moieties of the polyurethane prepolymer, that is, between polypropylene glycol and ethylene oxide/propylene oxide random copolymer has an influence on absorptivity of the dressing. More accurately, the higher the content of the ethylene oxide/propylene oxide random copolymer is, the larger the absorptivity of the dressing is.

Pore size of the wound-contacting layer of the dressing can, as apparent from the data of Examples 8 and 9, be controlled through changing a mixing ratio of surfactants, and as shown in the data of Example 10, through changing the mold temperature. When the pore size of the wound-contacting layer is less than 60 μm, time required for wound healing is reduced thanks to no contact of the dressing to the wound bed, providing an excellent wound healing effect.

Turning now to the toxicity test results, the viable cells in contact with the polyurethane foam dressing (Example 2) of the present invention were found to be reduced by 6 %, 30 % and 47 %, in comparison with the control cells, at day 1, 3 and 5, respectively, but show far greater viability than dressings of Comparative Examples 1 and 2. Additionally, the dressings of Comparative Examples 1 and 2 damaged the freshly regenerated tissues upon changing, and showed the wound healing mechanism depending only on wound contraction. In contrast, the polyurethane foam dressing of the present invention caused no injury to the tissue of the wound bed upon changing and, under the polyurethane foam dressing, re-epithelialization occurred together with the contraction of the wound, minimizing the scar formation after healing.

As described hereinbefore, the multilayered foam dressing of the present invention is a 3-ply structure consisting of an outer protective film layer 10-90 μm thick, an intermediate absorbent foam layer containing numerous open cells with an average diameter of 80-400 μm, and a wound-contacting layer 0.1-200 μm thick with numerous pores having an average diameter less than 60 μm. With this characterized structure, the polyurethane foam dressing of the present invention shows high moisture permeability, high absorptivity, and low wound adherency, as well as defending against the infiltration of bacteria and impurities. Additionally, the dressing of the present invention enjoys the advantage of minimizing scar formation after wound healing and a higher rate of healing.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

**Claims**

1. A microporous foam dressing, having a three-layer structure consisting of an outer protective film layer 10-90 μm thick, an intermediate absorbent foam layer containing numerous open cells with an average diameter of 80-400 μm, and a wound-contacting layer with numerous micropores having an average diameter less than 60 μm.

2. The microporous foam dressing as set forth in claim 1, wherein the microporous foam dressing is 1-7 mm in thickness.

3. The microporous foam dressing as set forth in claim 1, wherein the intermediate absorbent foam layer is 0.1-0.4 g/cm$^3$ in density.

4. The microporous foam dressing as set forth in claim 1, wherein the micropores comprise a structure opening into the exterior and another structure separated from the exterior by being covered with a thin cell window.

5. A method for manufacturing a microporous foam dressing, comprising the steps of:

   treating one side of a mold with an outer protective film layer,
   mixing 40-75 wt% of a polyurethane prepolymer, 15-45 wt% of a foaming agent, 5-35 wt% of a crosslinking agent, and 0.5-15 wt% of additives comprising a surfactant, a humectant, a releasing agent, an antibacterial agent, pigment, and a cell growth factor, and
   expanding the mixture in said mold.

FIG. 1

FIG. 2

**European Patent Office**

**EUROPEAN SEARCH REPORT**

Application Number

EP 02 01 4474

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | US 5 445 604 A (LANG STEPHEN M) 29 August 1995 (1995-08-29) * column 2, line 6 - line 14 * * column 2, line 62 - line 65 * * column 4, line 3 - line 8 * * column 5, line 25 - line 28 * * column 5, line 57 - line 63 * --- | 1-3 | A61F13/02 A61F13/00 C08J9/34 |
| A | US 5 098 621 A (HERMANN PAUL F) 24 March 1992 (1992-03-24) * column 9, line 49 - line 55; claims; examples * ----- | 5 | |
| | | | TECHNICAL FIELDS SEARCHED (Int.Cl.7) A61F C08J |

The present search report has been drawn up for all claims

| Place of search | Date of completion of the search | Examiner |
|---|---|---|
| THE HAGUE | 27 January 2003 | Mirza, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

**EP 1 374 812 A1**

**ANNEX TO THE EUROPEAN SEARCH REPORT
ON EUROPEAN PATENT APPLICATION NO.** EP 02 01 4474

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2003

| Patent document cited in search report | | Publication date | Patent family member(s) | | Publication date |
|---|---|---|---|---|---|
| US 5445604 | A | 29-08-1995 | AT | 14523 T | 15-08-1985 |
| | | | AT | 16345 T | 15-11-1985 |
| | | | AU | 552887 B2 | 26-06-1986 |
| | | | AU | 8037582 A | 19-08-1982 |
| | | | CA | 1174447 A1 | 18-09-1984 |
| | | | DE | 3264978 D1 | 05-09-1985 |
| | | | DE | 3267220 D1 | 12-12-1985 |
| | | | DK | 63882 A ,B, | 14-08-1982 |
| | | | EP | 0059048 A1 | 01-09-1982 |
| | | | EP | 0059049 A1 | 01-09-1982 |
| | | | IE | 52669 B1 | 20-01-1988 |
| | | | NZ | 199684 A | 20-03-1985 |
| | | | US | 4995382 A | 26-02-1991 |
| | | | US | 4860737 A | 29-08-1989 |
| | | | US | 5147338 A | 15-09-1992 |
| | | | US | 4753231 A | 28-06-1988 |
| | | | GB | 2093702 A ,B | 08-09-1982 |
| | | | GB | 2093703 A ,B | 08-09-1982 |
| | | | JP | 1654159 C | 13-04-1992 |
| | | | JP | 3013902 B | 25-02-1991 |
| | | | JP | 57153644 A | 22-09-1982 |
| | | | ZA | 8200933 A | 23-02-1983 |
| | | | AU | 6758990 A | 31-05-1991 |
| | | | WO | 9106295 A1 | 16-05-1991 |
| US 5098621 | A | 24-03-1992 | US | 4619948 A | 28-10-1986 |
| | | | US | 4828542 A | 09-05-1989 |

For more details about this annex : see Official Journal of the European Patent Office, No. 12/82

13